# EUROPEAN PATENT SPECIFICATION

(11) **EP 3 127 534 B1**
(45) Date of publication and mention of the grant of the patent: **20.09.2017**
(21) Application number: 15461556.1
(22) Date of filing: 28.08.2015
(51) Int. Cl.: A61K 8/97, A61Q 19/00

(54) **OIL/WATER COSMETIC EMULSION**
KOSMETISCHE ÖL/WASSER-EMULSION
ÉMULSION COSMÉTIQUE HUILE/EAU

(30) Priority: 07.08.2015 PL 41341615
(43) Date of publication of application: 08.02.2017
(73) Proprietor: Zachodniopomorski Uniwersytet Technologiczny w Szczecinie, 70-310 Szczecin (PL)
(72) Inventor: Andrys, Dominika, 71-424 Stargard Szczeci ski (PL); Kulpa, Danuta, 71-424 Szczecin (PL)
(74) Representative: Zawadzka, Renata

(56) References cited:
- CN-A- 1 586 176
- PL-B1- 187 715
- LAPPIN G J ET AL: "Biotransformation of monoterpenoids by suspension cultures of Lavandula angustifolia", PHYTOCHEMISTRY, PERGAMON PRESS, GB, vol. 26, no. 4, 1 January 1987 (1987-01-01), pages 995-997, XP026621245, ISSN: 0031-9422, DOI: 10.1016/S0031-9422(00)82334-1 [retrieved on 1987-01-01]
- PRASAD ARCHANA ET AL: "Genetic fidelity of long-term micropropagatedLavandula officinalisChaix.: an important aromatic medicinal plant", PLANT CELL, TISSUE AND ORGAN CULTURE, SPRINGER, NL, vol. 120, no. 2, 11 October 2014 (2014-10-11), pages 803-811, XP035435928, ISSN: 0167-6857, DOI: 10.1007/S11240-014-0637-7 [retrieved on 2014-10-11]
- MIGUEL CARLOS SÁNCHEZ-GRAS ET AL: "Micropropagation of Lavandula latifolia through nodal bud culture of mature plants", PLANT CELL, TISSUE AND ORGAN CULTURE., vol. 45, no. 3, 1 June 1996 (1996-06-01), pages 259-261, XP055326027, NL ISSN: 0167-6857, DOI: 10.1007/BF00043639
- NITZSCHE A ET AL: "Chemical and biological characterization of cinnamic acid derivaties from cell cultures of lavender (Lavandula officinalis) induced by stress and Jasmonic acid", MEDICINAL & AROMATIC PLANTS ABSTRACTS, SCIENTIFIC PUBLISHERS, NEW DELHI - INDIA, vol. 26, no. 6, 1 December 2004 (2004-12-01), XP018002398,
- EL-KELTAWI N E ET AL: "Influence of foliar applied cytokinins on growth and essential oil content of several members of the lamiaceae", PHYTOCHEMISTRY, PERGAMON PRESS, GB, vol. 26, no. 4, 1 January 1987 (1987-01-01), pages 891-895, XP026621223, ISSN: 0031-9422, DOI: 10.1016/S0031-9422(00)82312-2 [retrieved on 1987-01-01]

## Description

Subject of the invention is an oil/water cosmetic emulsion containing essential oil.

In recent years, cosmetics sector has been looking for new solutions on collecting and using natural biologically active substances. They are looked for in a large compound group of plant secondary metabolites. Noteworthy are essential oils collected from various medicinal plants, especially from lavender (*Lavandula angustifolia*)*.* Composition of lavender essential oils differ between cultivars. Essential oil obtained from lavender has the most diverse composition within the genus *Lavandula* (Boelens, 1995). One of the key possibilities of using lavender essential oil is use in various products as preservatives, primarily in the cosmetics, pharmaceutical as well as food industry. Plant secondary metabolites are produced in very low amounts and only during certain plant developmental stages. They can be also produced under the effect of external environmental stressors.

Due to the fact that under natural conditions collection of secondary metabolites is limited by the aforementioned factors used is a method of *in vitro* tissue cultures which allows very efficient production under sterile, controlled conditions. Elicitors which are stressogenic substances are often added to culture medium on which plants are grown. For instance, jasmonic acid can be used for this purpose. It allows obtaining essential oils with higher concentrations (Gundlach *et al.* 1991) and more diverse composition, containing greater amount of desired ingredients with antimicrobial and antioxidant effect (Devi *et al*., 2011).

From the Polish patent PL 187715 known is a body cleaning agent consisting of ethoxylated lauryl alcohol sulfate sodium salt in a proportion 10 to 14% by weight, coconut oil fatty acids amidoalkyl betaine in a proportion 3 to 6% by weight, coconut oil acids diethanolamide in a proportion 0.5 to 2% by weight, polyoxyethylene ester and coconut acids in a proportion 0.5 to 4% by weight, 5-ureidohydantoin in a proportion 0.1 to 5% by weight, preservative, pH regulator to the value from 5 to 7.5, viscosity adjuster and water. Additionally, the agent contains mixture of natural essential oils and ethylene oxide adduct with hydrogenated castor oil in a proportion 0.3% to 3% by weight, and the mixture of essential oils contains at least one oil from the group of tea tree oil, cananga oil, cedar oil, neroli oil, sandalwood oil, ylang-ylang oil, hyssop oil and cypress oil in a proportion 1 to 60% by weight and at least one oil from the group including lavender oil, rosemary oil, clary sage oil, rose oil, rose tree oil, *Pelargonium graveolens* oil, lemon oil, basil oil in a proportion 1 to 60% by weight. From the Polish patent application P. 402321 known is a preparation for hand skin care that contains essential oils, mineral and/or plant greasing oils and moisturizing substances and possibly other ingredients used in cosmetic preparations. It is characterized by containing 0.5 to 2.5% by weight of mixture of at least three natural essential oils selected from the following oils: tea tree, *Pelargonium graveolens*, lavender, rosemary, Roman chamomile, while mineral and/or plant greasing oils in a proportion 0.5 to 15.0% by weight and moisturizing substances in a proportion from 0.5 to 5.0% by weight in relation to the preparation weight. From the Polish patent application P.401135 known is a method of production cosmetic agents for skin care, in which mixed are measured ingredients until cosmetic mixture is obtained; then the obtained mixture is placed in individual containers and packed in packaging. This method is characterized by the fact that the cosmetic mixture is prepared by mixing its ingredients: 0.05%-5% by weight of nutritional substances, 5%-20% by weight of fatty substances, 2.5%-15% by weight of alcohols, 5%-20% by weight of emollients, 1.5%-20% by weight of surfactants, 1%-10% by weight of moisturizing agents, 0.1%-10% by weight of UV protectants, 0.02%-1.00% by weight of preservatives, 0.1%-2,5% by weight of viscosity adjusters, 0.05-0.0 bn 15% by weight of complexing agents, 1.5%-70% by weight of the component from lavender flowers with high essential oil content and water as complement to 100% by weight. Components of the mixture are mixed in several stages: in the first stage at 75°C mixed is phase I including components resistant to the effects of temperature of above 75°C, phase II including fatty components resistant to the effects of temperature of above 75°C and phase III including thickening components and stabilizers, after that emulsification homogenisation of the components of phases I, II and III is conducted at over 70°C, then the mixture is cooled to below 50°C, then cross-linking substances are added to the mixture, after which it is cooled to below 40°C and active substances sensitive to temperature above 40°C are added, then the mixture is cooled to 25°C and the component from lavender flowers cooled to 15°C is added and while continuing mixing the mixture is cooled to temperature in the range between 20°C and 25°C. Then, the mixed and cooled mixture is placed in airtight containers, in which the mixture is matured for 7 days at a temperature below 20°C, after which period the mixture is placed in individual containers.

Oil/water cosmetic emulsion, according to the invention, containing essential oil, plant oils, moisturizing substances is characterized by containing 0.1 to 1% by weight of lavender essential oil which originates from the cultivar 'Munstead' of lavender *(Lavandula angustifolia)* grown in *in vitro* tissue cultures on MS culture medium enriched with 2 mg·dm⁻³ kinetin, 0.2 mg·dm⁻³ indolyl-3-acetic acid and 0.2 mg·dm⁻³ jasmonic acid. The method of lavender multiplication was described in the Polish patent application P.408906. Cosmetic emulsion is produced using known methods. The cosmetic emulsion contains aqueous phase in a proportion 52.2 to 90% by weight, oil phase in a proportion 9.5 to 46.8% by weight. For the preparation of the cosmetic emulsion used were cosmetic ingredients found in the International Nomenclature of Cosmetic Ingredients (INCI). Oil phase consisted of glyceryl monostearate in a proportion 0.5-10% by weight, *Rubus idaeus* seed oil in a proportion 0.5-10% by weight, *Fragaria ananassa* seed oil in a proportion 0.5-10% by weight, *Citrullus vulgaris* seed oil in a proportion 0.5-10% by weight, *Calophyllum inophyllum* seed oil in a proportion 0.5-10% by weight, *Oendhera biennis* seed oil in a proportion 0.5-10% by weight and cetyl alcohol in a proportion 0.5-10% by weight. Aqueous phase consisted of water in a proportion 50-80% by weight, glycerol in a proportion 2-6% by weight, xanthan gum in a proportion 0.2-4% by weight. Emulsion may also contain other components, e.g. in oil phase *Borago officinalis* seed oil, *Carthamus tinctorius, Helianthus annuus* seed oil, *Hippophae rhamnoides* fruit oil, *Junglans regia* seed oil, *Linum usitatissiumum* seed oil, *Rosa canina* (fruit) oil, *Triticum aestivum, Vitis vinifera* seed oil.

The advantage of the solution consists in the fact that growing plants on MS culture medium containing in its composition 2 mg·dm⁻³ kinetin, 0.2 mg·dm⁻³ indolyl-3-acetic acid and 0.2 mg·dm⁻³ jasmonic acid allows to increase production of lavender essential oil and obtain more diverse composition of the oil and therefore its better preservative, antimicrobial and antioxidant characteristics. Thus, the oil obtained in this way is a natural preservative (in antimicrobial and antioxidative terms) and allows elimination the use of chemical preservatives. Emulsion from the invention has a positive effect on fibroblast multiplication and has a favorable effect on the quality of the skin.

The invention is presented more closely in the following preparation examples. Cosmetic emulsion stability was tested according to the examples for the period of 3 months. The cosmetic emulsion was subjected to a 4-stage stability evaluation method:
- exposure of the emulsion to high temperature-the emulsion was kept for 3 months at 35°C. After this period the emulsion was stable;
- temperature changes-the emulsion was subjected to a cycle of temperature changes. Initially the emulsion was placed at temperature -20°C for 24h, then the emulsion was placed in room temperature for 24h. The cycle was repeated three times. After the experiment the emulsion was stable;
- centrifuge use-the cosmetic emulsion was placed into Eppendorf test tubes and centrifuged for 20 minutes at 3000 rpm. After centrifuging the emulsion was stable;
- light exposure-the cosmetic emulsion in a closed jar was subjected to direct exposure to daylight. At the same time, a second jar with the cosmetic emulsion was protected with aluminum foil against exposure to light. After 3 months both emulsions were stable.

### Example I

The cosmetic emulsion is prepared from the following ingredients:
- oil phase in a proportion 46.8% by weight, which consists of: glyceryl monostearate in a proportion 9% by weight, *Rubus idaeus* seed oil in a proportion 10% by weight, *Fragaria ananassa* seed oil in a proportion 9% by weight, *Citrullus vulgaris* seed oil in a proportion 7% by weight, *Calophyllum inophyllum* seed oil in a proportion 5% by weight, *Oendhera biennis* seed oil in a proportion 4% by weight and cetyl alcohol in a proportion 2.8% by weight
- aqueous phase in a proportion 52.2% by weight which consists of: water in a proportion 50% by weight, glycerol in a proportion 2% by weight, xanthan gum in a proportion 0.2% by weight,
- lavender oil in a proportion 1% by weight.

In order to obtain the emulsion the oil phase and aqueous phase are heated in water bath to 80°C and melt the ingredients. Then the oil phase is added to the aqueous phase and mixed using homogenizer at 200 rpm. After cooling the cosmetic emulsion to approximately 35°C lavender essential oil is added.

### Example II

Cosmetic emulsion prepared as in the Example I, but it is prepared from the following ingredients:
- oil phase in a proportion 9.5% by weight, which consists of: glyceryl monostearate in a proportion 4% by weight, *Rubus idaeus* seed oil in a proportion 2% by weight, *Fragaria ananassa* seed oil in a proportion 0.8% by weight, *Citrullus vulgaris* seed oil in a proportion 0.7% by weight, *Calophyllum inophyllum* seed oil in a proportion 0.5% by weight, *Oendhera biennis* seed oil in a proportion 0.5% by weight and cetyl alcohol in a proportion 1% by weight
- aqueous phase in a proportion 90% by weight which consists of: water in a proportion 80% by weight, glycerol in a proportion 6% by weight, xanthan gum in a proportion 4% by weight
- lavender oil in a proportion 0.5% by weight.

### Example III

Cosmetic emulsion prepared as in the Example I, but it is prepared from the following ingredients:
- oil phase in a proportion 23.9% by weight, which consists of: glyceryl monostearate in a proportion 5% by weight, *Rubus idaeus* seed oil in a proportion 5% by weight, *Fragaria ananassa* seed oil in a proportion 4.5% by weight, *Citrullus vulgaris* seed oil in a proportion 3.4% by weight, *Calophyllum inophyllum* seed oil in a proportion 2.5% by weight, *Oendhera biennis* seed oil in a proportion 2% by weight and cetyl alcohol in a proportion 1.5% by weight
- aqueous phase in a proportion 76% by weight, which consists of: water in a proportion 70% by weight, glycerol in a proportion 4% by weight, xanthan gum in a proportion 2% by weight
- lavender oil in a proportion 0.1% by weight.

### Example IV

Cosmetic emulsion prepared as in the Example I, but it is prepared from the following ingredients:
- oil phase in a proportion 28.5% by weight, which consists of: glyceryl monostearate in a proportion 8% by weight, *Rubus idaeus* seed oil in a proportion 8% by weight, *Fragaria ananassa* seed oil in a proportion 4% by weight, *Citrullus vulgaris* seed oil in a proportion 3% by weight, *Calophyllum inophyllum* seed oil in a proportion 2% by weight, *Oendhera biennis* seed oil in a proportion 2% by weight and cetyl alcohol in a proportion 1.5% by weight
- aqueous phase in a proportion 71.4% by weight, which consists of: water in a proportion 67.69% by weight, glycerol in a proportion 3.31% by weight, xanthan gum in a proportion 0.4% by weight
- lavender oil in a proportion 0.1% by weight.

## Claims

1. Oil/water cosmetic emulsion containing essential oil, plant oils, moisturizing substances, **characterized by** containing 0.1 to 1% by weight of lavender essential oil from the cultivar 'Munstead' of lavender (*Lavandula angustifolia)* grown in *in vitro* tissue cultures on MS culture medium enriched with 2 mg·dm⁻³ kinetin, 0.2 mg·dm⁻³ indolyl-3-acetic acid and 0.2 mg·dm⁻³ jasmonic acid.

2. Cosmetic emulsion according to the claim 1, **characterized by** containing aqueous phase in a proportion 52.2 to 90% by weight, oil phase in a proportion 9.5 to 46.8% by weight.

3. Cosmetic emulsion according to the claim 2, **characterized by** the oil phase consisting of glyceryl monostearate in a proportion 0.5-10% by weight, *Rubus idaeus* seed oil in a proportion 0.5-10% by weight, *Fragaria ananassa* seed oil in a proportion 0.5-10% by weight, *Citrullus vulgaris* seed oil in a proportion 0.5-10% by weight, *Calophyllum inophyllum* seed oil in a proportion 0.5-10% by weight, *Oendhera biennis* seed oil in a proportion 0.5-10% by weight and cetyl alcohol in a proportion 0.5-10% by weight.

4. Cosmetic emulsion according to the claim 2, **characterized by** the aqueous phase consisting of water in a proportion 50-80% by weight, glycerol in a proportion 2-6% by weight, xanthan gum in a proportion 0.2-4% by weight.

## Patentansprüche

1. Öl/Wasser-kosmetische Emulsion, die ätherisches Öl, pflanzliche Öle, feuchtigkeitsspendende Substanzen enthält, **dadurch gekennzeichnet, daß** sie 0,1 bis 1 Gewichtsprozent Lavendel ätherisches Öl aus der Sorte "Munstead" von Lavendel *(Lavandula angustifolia*) enthält, die in *in vitro* Gewebekulturen auf MS gezüchtet wird Kulturmedium, angereichert mit 2 mg·dm⁻³ Kinetin, 0,2 mg·dm⁻³ Indolyl-3-essigsäure und 0,2 mg·dm⁻³ Jasmonic säure.

2. Kosmetische Emulsion nach Anspruch 1, **dadurch gekennzeichnet, daß** sie wäßrige Phase in einem Anteil von 52,2- 90 Gewichtsprozent, Ölphase in einem Anteil von 9,5-46,8 Gewichtsprozent enthält.

3. Kosmetische Emulsion nach Anspruch 2, **dadurch gekennzeichnet** die Ölphase bestehend aus Glyceryl monostearat in einem Anteil von 0,5-10 Gewichtsprozent, *Rubus idaeus* Samenöl in einem Anteil von 0,5-10 Gewichtsprozent, *Fragaria Ananassa* Samenöl in einem Anteil von 0,5-10 Gewichtsprozent, *Citrullus vulgaris* Samenöl in einem Anteil von 0,5-10 Gewichtsprozent, *Calophyllum Inophyllum* Samenöl in einem Anteil von 0,5-10 Gewichtsprozent, *Oendhera Biennis* Samenöl in einem Anteil von 0,5-10 Gewichtsprozent und Cetyl alkohol in einem Anteil von 0,5-10 Gewichtsprozent.

4. Kosmetische Emulsion nach Anspruch 2, **dadurch gekennzeichnet** die wässrige Phase, bestehend aus Wasser in einem Anteil von 50-80 Gewichtsprozent, Glycerin in einem Anteil von 2-6 Gewichtsprozent, Xanthan gummi in einem Anteil von 0,2-4 Gewichtsprozent.

## Revendications

1. Emulsion cosmétique huile / eau contenant de l'huile essentielle, des huiles végétales, des substances hydratantes, **caractérisée en ce qu'**elle contient 0,1 à 1% en poids d'huile essentielle de lavande du cultivar 'Munstead' de lavande (*Lavandula angustifolia*) cultivée dans des cultures de tissus *in vitro* sur MS milieu enrichi en 2 mg·dm⁻³ kinetine, 0,2 mg·dm⁻³ indolyl-3-acétique et 0,2 mg·dm³ acide jasmonique.

2. Emulsion cosmétique selon la revendication no. 1, **caractérisée par** la présence d'une phase aqueuse dans une proportion de 52,2 à 90% en poids, en phase huile dans une proportion de 9,5 à 46,8% en poids.

3. Emulsion cosmétique selon la revendication no. 2, **caractérisé par** la phase huileuse consistant en la monostéarate de glycéryle dans une proportion de 0,5 à 10% en poids, l'huile de graines *Rubus idaeus* dans une proportion de 0,5 à 10% en poids, l'huile de graine *Fragaria ananassa* dans une proportion de 0,5 à 10% en poids, *Citrullus vulgaris* Huile de graines dans une proportion de 0,5 à 10% en poids, huile de graines de *Calophyllum inophyllum* dans une proportion de 0,5 à 10% en poids, huile de graines *Oendhera biennis* dans une proportion de 0,5 à 10% en poids et alcool cétylique dans une proportion de 0,5 à 10% par poids.

4. Emulsion cosmétique selon la revendication no. 2, **caractérisé en ce que** la phase aqueuse est constituée d'eau dans une proportion de 50 à 80% en poids, de glycerol dans une proportion de 2 à 6% en poids, de gomme de xanthane à raison de 0,2 à 4% en poids.
